# EUROPÄISCHE PATENTSCHRIFT

(11) **EP 0 693 275 B1**
(45) Veröffentlichungstag und Bekanntmachung des Hinweises auf die Patenterteilung: **22.04.1998**
(21) Anmeldenummer: 94112190.7
(22) Anmeldetag: 04.08.1994
(51) Int. Cl.: A61F 5/01, A61F 13/04

(54) **Vorfuss-Entlastungsschuh**
Shoe for relieving the fore-foot
Chaussure pour décharger l'avant-pied

(30) Priorität: 21.07.1994 DE 9411782 U
(43) Veröffentlichungstag der Anmeldung: 24.01.1996
(73) Patentinhaber: Podia Trade Heil- und Hilfsmittel GmbH & Co. KG, 21337 Lüneburg (DE)
(72) Erfinder: Prahl, Jan, D-21379 Rullstorf (DE)
(74) Vertreter: Richter, Werdermann & Gerbaulet

(56) Entgegenhaltungen:
- EP-A- 0 248 964
- WO-A-92/08382
- US-A- 3 847 147
- US-A- 4 546 557

## Beschreibung

Die Erfindung betrifft einen Vorfuß-Entlastungsschuh mit einem in einer Seitenansicht oder einem vertikalen Längsquerschnitt im wesentlichen dreieckigen Sohlenteil mit nach hinten abnehmender Dicke, das vorn vor dem Mittelfußbereich des Trägers endet.

Ein solcher Vorfuß-Entlastungsschuh ist aus der US-A 45 46 557 bekannt.

Chirurgische Eingriffe im Vorfußbereich, insbesondere bei Hallux valgus-Operationen erfordern eine postoperative Schonung des (noch) nicht belastbaren Vorderfußes. Will der Patient während dieser Zeit nicht auf Krücken laufen, bietet sich der eingangs genannte Entlastungsschuh an, dessen schräggestelltes Fußbett in Verbindung mit dem vor dem Mittelfußbereich endenden Sohlenteil sicherstellt, daß der Patient ohne Vorfußbelastung auftreten und auch gefahrlos abrollen kann. Der Schuh nach der genannten US-Patentschrift besitzt eine kompressible Sohle mit einer von hinten nach vorne wachsenden Kompressibilität. Diese Ausgestaltung soll dazu dienen, Stöße im Vorderbereich des Schuhs besser absorbieren zu können.

Es ist Aufgabe der vorliegenden Erfindung, den Schuh dahingehend weiterzuentwickeln, daß er ohne Schädigung des Laufblockes längere Tragzeiten bzw. Benutzungszeiten ermöglicht. Hierbei soll ein möglichst guter Tragkomfort sichergestellt sein.

Diese Aufgabe wird durch den Vorfuß-Entlastungsschuh mit den im Anspruch 1 beschriebenen Merkmalen gelöst. Erfindungsgemäß besitzt dessen Sohlenteil ein Hohlprofil mit im wesentlichen vertikalen Stützen zur Verbindung der vollflächig ausgebildeten Basis und dem vollflächigen, an das Fußbett angrenzenden Oberkörper. Die Stützen weisen zumindest im mittleren Bereich in längsaxialer Richtung zur Basis hin einen zunehmenden Durchmesser oder einen zunehmenden Horizontalquerschnitt auf. Es hat sich herausgestellt, daß ein homogener Sohlenteilblock bzw. Schaumblock für die Abrollfunktion ungeeignet ist, da hierdurch zu große Schubkräfte auf das Hüftgelenk übertragen werden. Auf das Sohlenteil wirken halbelastisch Schub- und Abrollkräfte, die nur bei gegebener Horizontalelastizität eine ausreichende bzw. verbesserte Lebensdauer des Vorfußentlastungsschuhs gewährleisten. Diesem Ziel wird durch die Merkmalskombination nach Anspruch 1 Rechnung getragen.

Weiterbildungen der Erfindung sind in den Ansprüchen 2 bis 9 beschrieben. So weisen die Stützen vorzugsweise eine Zylinderstumpfform auf, d. h. sie verbreitern sich nach unten hin konisch. Die Mittellängsachse der einzelnen Stützen ist um einen Winkel zwischen 10° und 20° nach hinten geneigt, d. h. etwa in einem Winkel, daß die Stützen relativ zum Fußbett des Entlastungsschuhs bzw. dessen Sohlenteil-Oberkörper im wesentlichen senkrecht stehen. Die Längsachse dieses Oberkörpers (bzw. des Fußbettes) ist um 5° bis 15° gegenüber der Horizontalen oder der Basis geneigt.

Zur Verbesserung der Stoßabsorption ist die vordere Abschlußwand in einer Längsquerschnittansicht S-förmig gebogen. Die an die vordere Abschlußwand angrenzende Stütze besitzt nach einer weiteren Ausgestaltung der Erfindung über ihre gesamte Länge einen gleichbleibenden Querschnitt (von oben nach unten). Hiermit wird dem Umstand Rechnung getragen, daß die größte Bruchanfälligkeit des Entlastungsschuhs etwa im mittleren Teil des Sohlenteils bestanden hat, so daß eine Verstärkung der Stützen nach unten hin im Vorderbereich nicht erforderlich ist. Vorzugsweise erstrecken sich die Stützen über die gesamte jeweilige volle Breite der Sohle, d. h. von Seitenwand zu Seitenwand, wodurch sie als Querwände einzelne hintereinanderliegende Hohlräume voneinander abtrennen. Das so gestaltete Hohlraumprofil ist gegenüber einem Vollprofil dauerelastischer, materialsparend und dementsprechend leichter.

Um die Gangabwicklung des Trägers vom Zeitpunkt der Fersenberührung bis zur vorderen Abrollkante physiologisch besser zu gestalten, ist nach einer weiteren Ausgestaltung der Erfindung durch Wahl des Materials und/oder geometrische Ausbildung der Stützen die Elastizität (Weichheit) nach hinten, d. h. zum Fersenbereich zunehmend ausgebildet. Durch die Weicheinstellung des hinteren Teils entsteht beim Fersenauftritt eine zur Abrollung hin abnehmende Dämpfung, wodurch berücksichtigt wird, daß bei der Abrollung im vorderen Teil eine hohe Stabilität benötigt wird. Hierdurch wird vorteilhafterweise gewährleistet, daß die Zehen beim Abrollen weitergehend als bei einem Vollprofil vor einem Bodenkontakt geschützt sind.

Vorzugsweise liegt oberhalb einer von dem vorderen Sohlenfußpunkt zur Oberkante des hinteren Sohlenendes verlaufenden Diagonalebene ein härteres Material als unterhalb dieser Ebene vor.

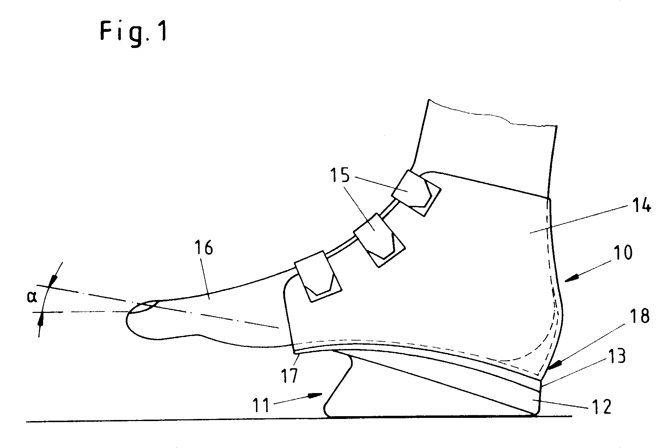

Ein Ausführungsbeispiel der Erfindung ist in den Zeichnungen dargestellt. Es zeigen
- Fig. 1: eine schematische Seitenansicht eines Vorfuß-Entlastungsschuhs,

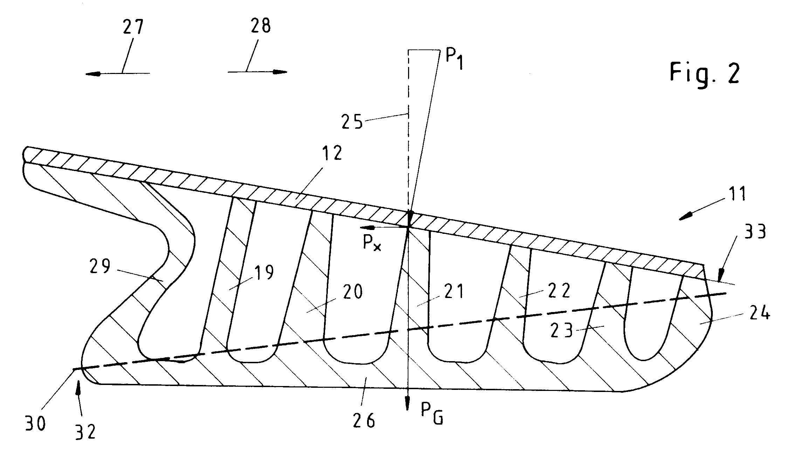

- Fig. 2: eine Detailansicht dieses Entlastungsschuhs im Bereich des Sohlenteils,
- Fig. 3a u. 3c: jeweils eine Stütze entsprechend der Ausgestaltung nach Fig. 2 und
- Fig. 3b: ein Drehmomentdiagramm entlang der Längsachse der Stütze nach Fig. 3a.

Der Vorfußentlastungsschuh 10 besteht im wesentlichen aus einem Sohlenteil 11, gegebenenfalls einem Zwischenstück oder Oberkörper 12, der Teil eines Fußbettes 13 sein kann, sowie einem aus zwei mit dem Oberkörper bzw. dem Fußbett verbundenen Schalen 14 bestehenden Teil, die beispielsweise über Klettverschlüsse 15 verschlossen werden können. Die Schalen 14 können aus Leder, Kunststoff oder Textilgewebe bestehen. Der Vorfuß-Entlastungsschuh ist so geformt, daß der Vorfuß 16 frei liegt und zur Fußspitze hin angehoben wird, was durch die im wesentlichen dreieckige Kontur des Sohlenteils 11 bewirkt wird. Der Neigungswinkel α liegt regelmäßig zwischen 5° und 15°. Das Fußbett 13 endet mit seinem vorderen Punkt 17 vor dem Mittelfußbereich, der nicht belastet werden soll. Beim Gehen kann der Träger des Entlastungsschuhs 10 zunächst im Bereich der Ferse 18 den Schuh aufsetzen und nach vorne hin abrollen, ohne Gefahr laufen zu müssen, mit dem Vorfuß bzw. den Zehen den Boden zu berühren.

Anders als in Ausführungsformen nach dem Stand der Technik ist jedoch das Sohlenteil 11 nicht als homogener Körper ausgebildet, sondern besitzt eine wabenartige Hohlprofilstruktur mit vertikalen Stützen 19 bis 23. Zumindest im mittleren Bereich, d. h. im Bereich der Mittelsenkrechten 25, sind die Stützen 20, 21, 22 und 23 so ausgebildet, daß sie in längsaxialer Richtung zur Basis 26 hin einen zunehmenden Durchmesser bzw. einen zunehmenden Horizontalflächenquerschnitt aufweisen. Die Querschnittszunahme von dem Oberkörper 12 bis zur Basis 26 erhöht die Lebensdauer des Sohlenteils 11, weil den Schubkräften 27 und den Abrollkräften 28 jeweils eine größere Elastizität entgegengesetzt werden kann. Auf das Fußbett wirkende Kräfte P unter Index 1 teilen sich entsprechend dem Kraftparallelogramm in Vorschubkräfte P unter Index X und vertikal nach unten wirkende Kräfte P unter Index G auf. Durch die erfindungsgemäße Konstruktion wird die Nachgiebigkeit des Sohlenteils 11 im Bereich größerer Schub bzw. Abrollkräfte erhöht bzw. das Material in bodennahen Bereichen zur Aufnahme der Vertikalkräfte durch die Stützen verstärkt. Die vordere Stütze 19 kann über ihre Länge gesehen gleichmäßig dick bzw. breit ausgeführt sein, da nahe dem Vorfuß die Kräfte - anders als im Fersenbereich - geringer sind. Zur Stoßdämpfung und besseren Nachgiebigkeit ist die vordere Abschlußwand 29 S-förmig gebogen.

Zur Erhöhung des Lauf- bzw. Tragekomforts wird oberhalb der Diagonalebene 30 ein härteres Material, d. h. ein Material mit einer großen Federkonstanten und im darunter liegenden Bereich ein weiches Material mit einer kleineren Federkonstanten verwendet.

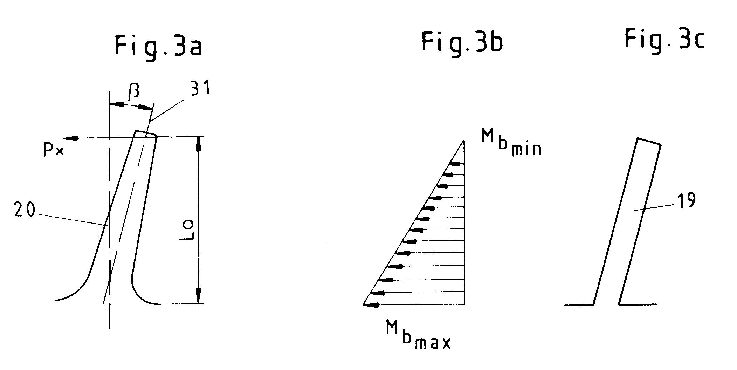

Wie aus Fig. 3a ersichtlich, ist die Stütze 20 in entsprechender Weise wie die übrigen Stützen 19 bis 23 nach hinten geneigt, wobei deren Längsachse 31 mit einer Vertikalen einen Winkel β zwischen 10° und 20° bildet. Die entlang der Stütze wirkenden Drehmomente, die sich entsprechend der Beziehung N unter Index B = P unter Index X x L ergeben, sind in Fig. 3b aufgetragen. Hieraus wird deutlich, daß die Drehmomentbelastung von oben nach unten hin in horizontaler Richtung zunimmt. In entsprechender Weise wird auch der Querschnitt der Stütze vergrößert. Dort, wo die angreifenden Drehmomente kleiner sind, nämlich nahe des Vorfußbereiches, kann die Ausbildung gemäß Fig. 3c gewählt werden.

## Patentansprüche

1. Vorfuß-Entlastungsschuh (10) mit einem in einer Seitenansicht oder einem vertikalen Längsquerschnitt im wesentlichen dreieckigen Sohlenteil (11) mit nach hinten abnehmender Dicke, das vorn vor dem Mittelfußbereich des Trägers endet, dadurch gekennzeichnet, daß das Sohlenteil (11) ein Hohlprofil mit im wesentlichen vertikalen Stützen (19 bis 23) zur Verbindung der vollflächig ausgebildeten Basis (26) und dem vollflächigen, an das Fußbett (13) angrenzenden Oberkörper (12) ist und daß die Stützen (19 bis 23) zumindest im mittleren Bereich in längsaxialer Richtung zur Basis (26) hin einen zunehmenden Durchmesser oder einen zunehmenden Horizontalflächenquerschnitt aufweisen.

2. Vorfuß-Entlastungsschuh nach Anspruch 1, dadurch gekennzeichnet, daß die Stützen (19 bis 23) eine Zylinderstumpfform aufweisen.

3. Vorfuß-Entlastungsschuh nach Anspruch 1 oder 2, dadurch gekennzeichnet, daß die Mittellängsachse (31) der Stützen (20) um einen Winkel β zwischen 10° bis 20° nach hinten geneigt ist.

4. Vorfuß-Entlastungsschuh nach einem der Ansprüche 1 bis 3, dadurch gekennzeichnet, daß die Längsachse des Oberkörpers (12) um α 5° bis 15° gegenüber der Horizontalen oder der Basis (26) geneigt ist.

5. Vorfuß-Entlastungsschuh nach einem der Ansprüche 1 bis 4, dadurch gekennzeichnet, daß die vordere Abschlußwand (29) in einer Längsquerschnittansicht S-förmig gebogen ist.

6. Vorfuß-Entlastungsschuh nach einem der Ansprüche 1 bis 5, dadurch gekennzeichnet, daß die an die vordere Abschlußwand (29) angrenzende Stütze (19) einen über ihre gesamte Länge gleichbleibenden Querschnitt aufweist.

7. Vorfuß-Entlastungsschuh nach einem der Ansprüche 1 bis 6, dadurch gekennzeichnet, daß sich die Stützen (19 bis 23) über die gesamte jeweilige volle Breite der Sohle (11) erstrecken.

8. Vorfuß-Entlastungsschuh nach einem der Ansprüche 1 bis 7, dadurch gekennzeichnet, daß durch Wahl des Materials und/oder durch geometrische Ausbildung der Stützen (19 bis 23) die Elastizität (Weichheit) nach hinten, d. h. zum Fersenbereich (18), zunimmt.

9. Vorfuß-Entlastungsschuh nach Anspruch 8, dadurch gekenzeichnet, daß oberhalb einer von dem vorderen Sohlenfußpunkt (32) zur Oberkante (33) des hinteren Sohlendes verlaufenden Diagonalebene (30) ein härteres Material als unterhalb dieser Ebene (30) vorliegt.

## Claims

1. Shoe for relieving the forefoot (10) with a, when seen in a side view or in a vertical longitudinal section, essentially triangular sole portion (11) of a thickness decreasing towards the rear, which ends anteriorly before the metatarsal area of the wearer.
characterized in that
the sole portion (11) is a hollow section with essentially vertical supports (19 to 23) for interconnecting the base (26) so as to cover the entire area and the full-surface upper body (12) adjacent to the foot bed (13) and in that the supports (19 to 23),
at least in the longitudinal axial direction towards the base (26), possess an increasing diameter or an increasing horizontal surface cross section.

2. Shoe for relieving the forefoot according to Claim 1, characterized in that
the supports (19 to 23) possess the configuration of a truncated cone.

3. Shoes for relieving the forefoot according to either Claim 1 or 2,
characterized in that
the center longitudinal axis (31) of the supports (20) is inclined towards the rear by an angle β of between 10° to 20°.

4. Shoee for relieving the forefoot according to any of Claims 1 to 3,
characterized in that
the longitudinal axis of the upper body (12) is inclined by the angle α 5° to 15° in relation to the horizontal or the base (26).

5. Shoe for relieving the forefoot according to any of Claims 1 to 4,
characterized in that
the front end wall (29), in a longitudinal cross-sectional view, is bent so as to form an "S".

6. Shoe for relieving the forefoot according to any of Claims 1 to 5,
characterized in that
the support (19) adjacent to the front end wall (29) possesses a uniform cross section over its entire length.

7. Shoe for relieving the forefoot according to any of Claims 1 to 6,
characterized in that
the supports (19 to 23) extend over the entire respective full width of the sole (11).

8. Shoes for relieving the forefoot according to any of Claims 1 to 7,
characterized in that,
by the selection of the material and/or by the geometric construction of the supports (19 to 23), the elasticity (softness) increases towards the rear, i.e. towards the calcaneal region (18).

9. Shoe for relieving the forefoot according to Claim 8,
characterized in that,
above a diagonal plane (30) proceeding from the front plantar foot point (32) towards the top edge (33) of the sole end, a harder material is present below this plane (30).

## Revendications

1. Chaussure pour décharger l'avant-pied (10) avec une partie semelle (11) substantiellement triangulaire dans une vue latérale ou dans une section longitudinale verticale avec une épaisseur qui diminue vers l'arrière, qui se termine à l'avant devant la zone médiane du pied du porteur, caractérisée en ce que la partie semelle (11) est un profil creux avec des appuis (19 à 23) essentiellement verticaux pour relier la base (26) configurée sur toute la surface et le corps supérieur (12) sur toute la surface, adjacent au coussinet de pied (13) et que les appuis (19 à 23) présentent, au moins dans la zone médiane dans le sens axial longitudinal, un diamètre croissant vers la base (26) ou une section de surface horizontale croissante.

2. Chaussure pour décharger le pied selon la revendication 1, caractérisée en ce que les appuis (19 à 23) présentent une forme de cylindre tronqué.

3. Chaussure pour décharger le pied selon la revendication 1 ou 2, caractérisée en ce que l'axe longitudinal médian (31) des appuis (20) est incliné vers l'arrière d'un angle β entre 10° et 20°.

4. Chaussure pour décharger le pied selon l'une des revendications 1 à 3, caractérisée en ce que l'axe longitudinal du corps supérieur (12) est incliné par rapport à l'horizontale ou à la base (26) d'α de 5° à 15°.

5. Chaussure pour décharger le pied selon l'une des revendications 1 à 4, caractérisée en ce que la paroi de fermeture avant (29) est courbée en forme de S dans une vue de section longitudinale.

6. Chaussure pour décharger le pied selon l'une des revendications 1 à 5, caractérisée en ce que l'appui (19) qui est adjacent à la paroi de fermeture avant (29) présente une section qui reste constante sur toute sa longueur.

7. Chaussure pour décharger le pied selon l'une des revendications 1 à 6, caractérisée en ce que les appuis (19 à 23) s'étendent sur toute la largeur complète respective de la semelle (11).

8. Chaussure pour décharger le pied selon l'une des revendications 1 à 7, caractérisée en ce que, par le choix de la matière et/ou par la configuration géométrique des appuis (19 à 23), l'élasticité (la souplesse) augmente vers l'arrière, c'est-à-dire vers la zone du talon (18).

9. Chaussure pour décharger le pied selon la revendication 8, caractérisée en ce qu'au-dessus d'un plan diagonal (30) qui va du point de base antérieur de la semelle (32) à l'arête supérieure (33) de l'extrémité arrière de la semelle il y a une matière plus dure qu'au-dessous de ce plan (30).
